# EUROPEAN PATENT SPECIFICATION

(11) **EP 1 434 996 B1**
(45) Date of publication and mention of the grant of the patent: **29.07.2009**
(21) Application number: 02767702.0
(22) Date of filing: 10.10.2002
(51) Int. Cl.: G01N 33/543

(54) **BINDING ASSAY USING A MAGNETIC FIELD**
BINDUNGSASSAY UNTER VERWENDUNG EINES MAGNETISCHEN FELDS
DOSAGE PAR LIAISON UTILISANT UN CHAMP MAGNETIQUE

(30) Priority: 10.10.2001 GB 0124341
(43) Date of publication of application: 07.07.2004
(73) Proprietor: Randox Laboratories Ltd., Crumlin, Co. Antrim BT29 4QY (GB)
(72) Inventor: LUXTON, Richard, William University of the West of, Bristol BS16 1QY (GB); HAWKINS, Peter University of the West of, Bristol BS16 1QY (GB)
(74) Representative: Jappy, John William Graham
(86) International application number: PCT/GB2002/004594
(87) International publication number: WO 2003/031977

(56) References cited:
- WO-A-01/14591
- WO-A-96/07101
- US-A- 5 445 971
- US-A- 5 558 839
- HAWKINS P ET AL: "MEASURING SYSTEM FOR THE RAPID DETERMINATION OF THE CONCENTRATION OF COATED MICROMETER-SIZED PARAMAGNETIC PARTICLES SUSPENDED IN AQUEOUS BUFFER SOLUTIONS" REVIEW OF SCIENTIFIC INSTRUMENTS, AMERICAN INSTITUTE OF PHYSICS. NEW YORK, US, vol. 72, no. 1, PART 1/2, January 2001 (2001-01), pages 237-242, XP000996937 ISSN: 0034-6748
- BERGGREN KRIZ C ET AL: "MAGNETIC PERMEABILITY MEASUREMENTS IN BIOANALYSIS AND BIOSENSORS" ANALYTICAL CHEMISTRY, AMERICAN CHEMICAL SOCIETY. COLUMBUS, US, vol. 68, no. 11, 1 June 1996 (1996-06-01), pages 1966-1970, XP000594183 ISSN: 0003-2700 cited in the application

## Description

### Field of the Invention

The present invention relates to a method for performing a binding assay, in particular an immunoassay, using magnetic particles as a label.

### Background to the Invention

Immunoassays are ligand binding assays where the specific recognition of an antibody to the specific binding site of an analyte is exploited. The immunoassays usually involve the immobilisation of a ligand (antibody, other protein, hapten etc.) to a solid phase that will, in turn, be recognised by the analyte to be determined. The use of specific labelled detecting agents allows the subsequent detection and quantitation of the analyte in an aqueous sample. The amount of analyte present in the sample is a function of the bound labelled detecting agent: inversely proportional in competitive assays and directly proportional in non-competitive assays.

In the past decade, there have been many changes in the design and format of the immunoassays, resulting in an increase in sensitivity. Many innovations have been in the use of different labels used to monitor the immune reaction. Radioisotope labels were used originally, but these were replaced subsequently with enzyme and fluorescent/luminescent labels. These changes overcame many of the disadvantages of isotopes in terms of stability, storage of labelled components and ease of handling. Current analytical immunoassay systems using fluorescent or similar markers require expensive specialised equipment and it is not practical to make them into hand-held systems. The only hand-held systems available at present use dipstick technology with colour labels. They are mainly used for pregnancy tests where the outcome of the test can only either be positive or negative. They are generally not suitable for use in tests where the actual concentration of a species is being determined.

The use of ferromagnetic particles as labels in an immunoassay has been described in two papers by Kriz et al (Biosensors and Bioelectronics, 1998; 13: 817-823 and Analytical Chemistry, 1996; 68: 1966-1970). However, in these techniques the ferromagnetic particles are detected by a simple measuring coil which is placed in a Maxwell bridge to allow measurement of the magnetic permeability of a sample to be made. The magnetic permeability is then used as an indication to determine the number of particles within the substance.

Hawkins et al, Review of Scientific instruments: American Institute of Physics. New York, US; Vol.72 no.1 part 1/2 pages 237-242 January 2001 discloses the determination of paramagnetic particle concentration in a buffer solution by detecting a change in resonant frequency in a measuring system comprising a coil in a parallel resonant circuit with a capacitor.

European Patent Application No. EP-A-01303319.6 is a co-pending application by the Applicant, the content of which is incorporated herein by reference. This describes an immunoassay method which uses magnetic particles as a label; the particles being determined by a measurement of resonant frequency.

### Summary of the Invention

The present invention is based on the surprising finding that kinetic measurements can be obtained during a binding assay when using magnetic particles as a label and applying a magnetic field at regular time intervals during the time course of the reaction.

According to a first aspect of the invention, a method for obtaining kinetic measurements during a binding assay comprises the steps of:
(i) bringing a target molecule into contact with its affinity partner to form a binding complex, wherein the target molecule is immobilised on a magnetic particle and the affinity partner is immobilised on a support material; and
(ii) determining the number of complexed magnetic particles during the assay, by exposing the complex to a magnetic field at regular time intervals during the assay and measuring the difference in the resonant frequency of a tuned circuit when the complex is exposed to the magnetic field and when it is not.

The method of the invention can be used to enhance the speed of a binding assay, e.g. an immunoassay. Without wishing to be bound by theory, this appears to be the result of bringing the respective binding partners together, into close proximity, allowing the binding reaction to take place more effectively.

### Description of the Figures

The invention is described with reference to the accompanying figures, wherein:
Figure 1 illustrates a side view (a) and top view (b) of a test strip, where (1) represents a strip of polyethylene terephthalate with the hydrophobic surface uppermost, (2) represents a plastic stick, (3) is a wax line and (4) is a sample/biological reagent;
Figure 2 shows a frequency generator, power supply and electromagnets;
Figure 3 shows electromagnets, including studs;
Figure 4 is a graphic representation of the effect of increasing field strength on the number of paramagnetic particles bound to the support;
Figure 5 is a graphic representation of a calibration curve showing the magnet field strength of an electromagnet for a given applied voltage;
Figure 6 is a graphic representation of binding in an assay for CK-MB;
Figure 7 is a graphic representation of an assay for CRP;
Figure 8 is a graphic illustration showing frequency change against time; and
Figure 9 is a graphic illustration showing a calibration curve for the analyte CKMB, established by calculating the frequency change during an assay.

### Description of the Invention

The method according to the invention may be applied to any form of binding reaction, where it is desirable to determine the presence or concentration of a particular analyte. A suitable binding reaction is a DNA hybridisation reaction. In this case, the target molecule and its affinity partner will be complementary DNA molecules. A polynucleotide may also be used to bind to other agents. For example, the polynucleotide may be an aptamer, used to bind to say, DNA binding proteins. Preferably the binding reaction is an immunoassay in which a specific antibody/antigen binding complex is formed. General methods for carrying out binding assays, including DNA hybridisation reactions and immunoassays, will be apparent to the skilled person. Suitable techniques for carrying out the binding assays are referenced in Sambrook et al, Molecular Cloning, A Laboratory Manual (1989), and Ausubel et al, Current Protocols in Molecular Biology (1995), John Wiley and Sons Inc.

With regard to an immunoassay, the molecule immobilised on the magnetic particle may bind to an affinity partner that is bound directly on a support material. Alternatively, the affinity partner may bind indirectly to the support, e.g. via a separate affinity for a molecule immobilised on the support. In this example, the affinity partner may be present in an aqueous sample, which is brought into contact with the support and the magnetic particles. The affinity partner binds to the molecule on the support and to the molecule on the magnetic particle, thereby acting as a bridge between the support and the particle. This embodiment is illustrated in Example 1 given below. Variations will be apparent to the skilled person.

Typically, the magnetic particles used are paramagnetic particles, although ferromagnetic or any other magnetic particles may also be used. Paramagnetic particles are available from commercial sources, and are typically 2.8 µm in diameter and consist of a core of paramagnetic material coated with a suitable polymer layer onto which a molecule that is used in the binding assay may be immobilised.

Typically, each magnetic particle will comprise a single molecule, thereby allowing a direct calculation of the concentration of the molecules to be made. Methods for the immobilisation of a molecule onto the magnetic particles will be apparent to the skilled person. A specific example is detailed in Example 1 provided below, and alternatives will be apparent. However, the particles may comprise more than one molecule, if desired. The magnetic particles will typically have a diameter of approximately 0.5 µm - 5 µm, more preferably 1 µm - 3 µm. Nanoparticles, typically in a range 5 nm to 300 nm in diameter, are also within the scope of the invention.

The binding assay will be carried out with the affinity partner immobilised on a suitable support material. Suitable support materials will be apparent to the skilled person, based on conventional binding assays, in particular immunoassays. Suitable support materials include glass, ceramic, plastic and silicon support materials. In a preferred embodiment, the support material is a polyester-based material. Typically, the support will be a flat, planar, surface, onto which the affinity partner will be immobilised using conventional methods known in the art, although other configurations may also be used. In a preferred embodiment, the support material is in the form of a microarray. A suitable microarray is disclosed in GB-A-2324866, the content of which is incorporated herein by reference.

The binding assay is carried out in the presence of an applied magnetic field. The magnetic field may be applied using apparatus known to the skilled person, in particular an electromagnet or a solenoid. The magnetic field may be applied constantly, or may be varied by applying an external signal such as a square, sine, or sawtooth waveform.

It has been found, surprisingly, that varying the applied magnetic field provides improved results compared to the constant application of the magnetic field. More particularly, varying the magnetic field produces the greatest decrease in reaction time, and improves the likelihood of further binding events occurring as the magnetic particles are not held firmly during the periods when the applied magnetic field strength is low, so they can move and expose more binding sites to the immobilised target proteins.

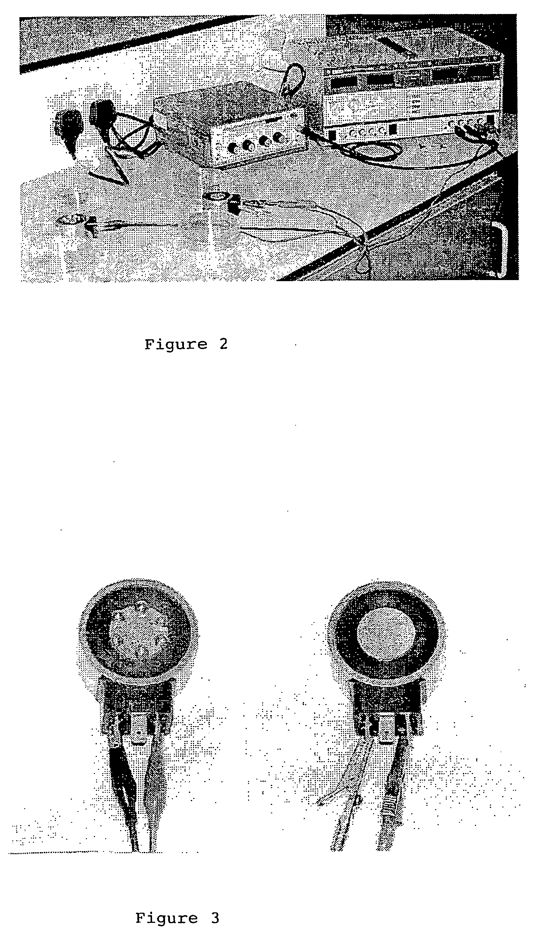

A suitable frequency of the magnetic field may be determined by the skilled person, through routine experimentation. A preferred frequency is less than 1 Hertz. In a preferred embodiment, the magnetic field is applied by means of a coiled electromagnet that comprises metal studs to focus the magnetic field. A suitable electromagnet comprising metal studs is shown in Figure 3. The support material should be placed over the studs on the electromagnet, to focus the magnetic field.

In a further preferred embodiment of the invention, one or more flat coils are used to apply the magnetic field. The coils can be deposited on the underside of the support material, i.e. the reverse side to that comprising the immobilised molecules (affinity partner). The coils may be bound to the support using thick film or thin film technology using a conducting material, e.g. metal, as will be appreciated by the skilled person. The resonant frequency of each coil is determined by its diameter, the number of turns of the coil and the material used in its construction. Single coils or multiple numbers of coils can be patterned on to the support for single or multi-analyte detection. The biological molecules (affinity partners) will be deposited on the reverse side of the support in such a position to overlie the coil(s). A series of binding events can then take place the support that will involve the incorporation of magnetic, paramagnetic or superparamagnetic material (micron or nanometre sized particles).

It has been found that, surprisingly, the assay can be performed to obtain kinetic measurements as opposed to making a final endpoint reading. Kinetic measurements are obtained by using a permanent magnet and switching this on and off.

In one embodiment, the assay is carried out using a system with two magnets; one underneath the support material to pull the magnetic particles to the surface of the support, and one above the support to pull non-bound particles away from the surface. The magnets may be switched on alternately, so that the particles are pulled in one direction at a time. A detection coil may be provided to interrogate the surface and measure the amount of magnetic material present. The upper and lower magnets may also be provided on an apparatus that allows the magnets to move towards or away from the support material. When the lower magnet is positioned close to the support, to exert the magnetic pull, the upper magnet is positioned away from the support, and does not exert its effect. The magnets can then be moved so that the opposite orientation is achieved. In this way, a varied magnetic field is applied to the assay components. Measuring the change in frequency when the upper magnet exerts its maximum effect, i.e. is close to the support, permits quantitative measurements to be made. The change in frequency is measured by subtracting the mean frequency of a blank (without analyte) from the mean frequency of the sample. If this is carried out at regular intervals, say every 2 minutes, over a set time course, it will be possible to establish calibration curves for each analyte under study.

The final step in the assay is the determination of the number of magnetic particles that are bound in a complex to the solid support. The magnetic particles are determined by measuring the difference in the resonant frequency of a tuned circuit when the complex comprising the bound magnetic particles is exposed to a magnetic field generated by a coil and when the complex is not exposed to a magnetic field. This aspect of the invention is described in the co-pending application EP-A-01303319.6.

The assay may be carried out in any suitable apparatus. For example, the support material may be a part of a test strip (diagnostic dipstick), which may be placed over a magnet when carrying out the diagnostic assay. Alternatively, the support material may be held within an appropriate sealed reaction vessel. The magnetic, paramagnetic or superparamagnetic particles may be placed in suspension in a suitable buffer within the reaction vessel, and the biological test sample, including the analyte to be measured, can then be introduced into the reaction vessel via an entry port. Suitable reaction vessels will be apparent to the skilled person.

The following Examples illustrate the invention.

### Example 1

### Preparation of plastic test strips

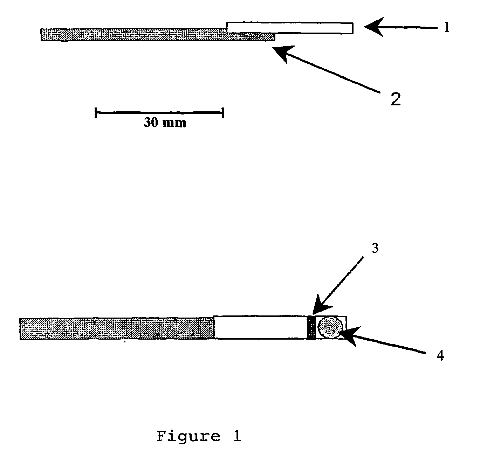

The support material used was polyethylene terephthalate (PT) transparencies, laser/copier type A, (Rank Xerox, UK). Strips of PT measuring 30 mm (length) x 5 mm (width) were glued to plastic sticks (ACS, UK) as shown in Figure 1a. The strips ofPT were oriented with the hydrophobic surface uppermost. In order to prevent the spread of applied reagents along the length of the PT test strip, in the direction of the plastic stick, a wax line was drawn across the PT as shown in Figure 1b.

### Magneto-immunoassay

*0.15 M phosphate buffered saline (PBS),* pH 7.2 was prepared as follows: 40 g sodium chloride, 7.2 g disodium hydrogen orthophosphate dihydrate, 1 g potassium chloride and 1 g potassium dihydrogen orthophosphate were all dissolved in 5 litres of distilled water. The pH was adjusted to 7.2 as required.

*Rabbit anti-human transferrin* (Dako AIS, Denmark) was diluted 1:1000 in phosphate buffered saline (PBS). 1% glutaraldehyde v/v diluted in PBS.

*Blocking solution:* 1% bovine serum albumin w/v (BSA) and 1 M glycine w/v dissolved in PBS.

*Human transferrin:* A stock solution of human holo-transferrin was prepared in PBS, giving a concentration of 1 mg ml⁻¹. The stock solution was then diluted in 1% BSA/PBS to a final concentration 1 µg ml⁻¹.

*Sheep anti-human transferrin:* Sheep anti-human transferrin was diluted 1:1000 in 1% BSA/PBS.

*Paramagnetic particles (PMP's):* Streptavidin coated M-280 Dynabeads, and magnetic particle concentrator (MPC) from Dynal, Norway.

*Washing buffer for PMP's:* 0.16 g sodium dihydrogen orthophosphate, 0.98 g disodium hydrogen orthophosphate dihydrate and 8.10 g sodium chloride dissolved in 1L distilled water. The pH was adjusted to 7.4 using hydrochloric acid or sodium hydroxide as required.

*Biotinylated donkey anti-sheep:* Biotinylated donkey anti-sheep for coating the paramagnetic particles was diluted in PBS.

*Final suspension buffer for PMP's:* PBS/ 1% bovine serum albumin/ 4% polyethylene glycol (PBS\BSA\PEG): 1% BSA w/v, 4% PEG w/v were dissolved in PBS.

'PL' series dual output laboratory power supply, Jupiter 500 function generator and 50 mm electromagnets (12 V) all from Farnell Electronic Company, Leeds, UK.

To aid the focusing of the magnetic field, studs were machined in-house to place on the electromagnets.

### Method

### i. Preparation of streptavidin PMP's

For each test strip 10 µl PMP's were re-suspended in a tube of washing buffer by vortexing for 1 minute, and then placed into a magnetic particle concentrator (MPC) for 2 minutes. This resulted in the paramagnetic particles being attracted and held to the side of the tube by the magnet in the MPC. The supernatant was removed. The procedure was repeated three times.

The coating procedure was performed as recommended in the Dynal handbook (Dynal, 1996). Streptavidin-paramagnetic particles were mixed with biotinylated donkey anti-sheep (1.5 µg ofprotein per 10⁷ paramagnetic particles), and incubated for 30 minutes at 4°C with bi-directional mixing (using the Dynal MX2 sample mixer, Dynal, Norway). Coated paramagnetic particles were then washed 4 times in 1% BSA/PBS using the MPC, and re-suspended in PBS\BSA\PEG.

### ii. Magneto-immunoassay

Solid-phase washing procedure: Unless otherwise stated the term 'washing' is used to describe the rinsing of solid phases with 3 x 3 ml distilled water followed by 1 x 3 ml of PBS using a pasteur pipette. Incubation temperature: Unless otherwise stated all incubations were performed at room temperature.

25 µl of rabbit anti-human transferrin was mixed with 10 µl of 1% glutaraldehyde (v/v) and applied to each test strip. Test strips were then incubated overnight at 4°C. After washing, the test strips were incubated in blocking solution for 1 hour. The test strips were then washed, and incubated with 20 µl of the human transferrin for 30 minutes. After washing, 30 µl sheep anti-human transferrin was applied and incubated on each test strip for 30 minutes, and the wash step was repeated. 10 µl (6.7 x 10⁵) coated paramagnetic particles were then applied to each test strip and incubated on an electromagnet accordingly (see below). A final wash step consisting of 4 x 3 ml distilled water + 0.02% Tween 20 using a pasteur pipette was performed to remove unbound paramagnetic particles.

A electromagnet was used to provide a magnetic field. This was either a permanent field, or was caused to vary, by applying an external signal as a square, sine or sawtooth waveform. In addition, metal studs were placed on the electromagnet to focus the magnetic field (Figure 3). Enhancement of the biological binding interaction was indicated by higher numbers of paramagnetic particles binding to the solid phase in a given time under given magnetic conditions. The following variables were investigated.
- Permanent magnetic field
- Square wave
- Sine wave
- Saw tooth wave
- Field strength
- Addition of "studs" to the electromagnet
- Offset from zero

Detection and quantitation of paramagnetic particles was carried out by the use of an optical microscope. This was used to eliminate further possible variables introduced by the magnetometer.

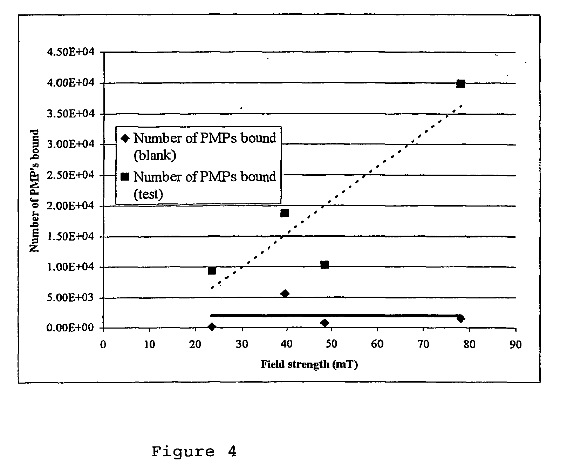

Initial experiments demonstrated that the binding of paramagnetic particles to a coated plastic surface using the analyte (transferrin) as a bridging agent occurred in a magnetic field. The number of particles binding to the surface was dependent on the applied field strength. Figure 4 shows increasing numbers of paramagnetic particles binding as a result of increasing the field strength of an applied constant magnetic field.

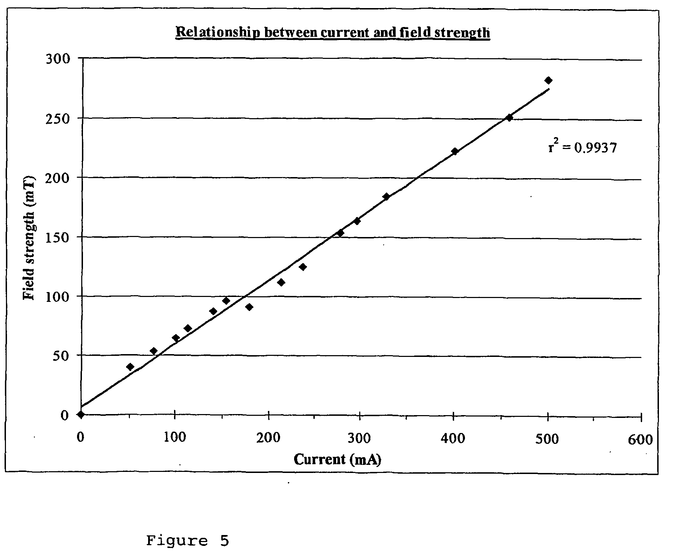

The magnet field strength was determined using a Hall probe and a calibration curve was obtained, relating the applied voltage to the magnet and the measured field strength. This is shown in Figure 5. Further experiments into the effect of varying the strength of the applied magnetic field gave varied results. Three types of waveform were investigated, square wave, sine wave and sawtooth wave. Initially, the frequency meter was adjusted to so that the amplitude of the waveform went from 0 volts to a voltage that corresponds to particular field strength.

The sawtooth waveform gave increasing numbers of particles with field strength for all incubation times, but overall this waveform gave the lowest number of paramagnetic particles binding. The square wave showed the highest number of paramagnetic particles binding, particularly with longer incubation times. The field strength appeared to have less of an effect, compared to sawtooth waveforms. Interestingly, a sine wave gave increased binding up to a particular field strength, which then decreased as the field strength increased. The best results were obtained using studs on the electromagnet, but this also gave high numbers of blanks.

The offset of the applied waveforms was also shown to be an important variable in the assay. The number of particles binding was greatly increased when the varying magnet field remained positive over the whole cycle.

### Example 2

### CK-MB Assay - using micron sized paramagnetic particles

The development of a direct, 5-minute assay for creatin-kinase MB subunit (CK-MB) was investigated. The capture antibody was a mouse monoclonal and the detecting antibody was a goat polyclonal, both directed against CK-MB. In this assay the goat antibody was immobilized to the support material.

A wide range of conditions for coating the support material was investigated to try and maximise the amount of protein immobilized. These included:
- Incubating overnight at 4°C.
- Incubating at room temperature for 4 hours.
- Irradiation of the solid phase for 15 minutes using a trans-illuminator prior
   to applying the protein.

All the above were with and without glutaraldehyde in the coating buffer and using a range of antibody dilutions: 1/200, 1/500 and 1/1000.

Following the preparation of the support material the assay was performed in a 2-step (indirect) manner and also as a single step (direct) assay. In the indirect assay the addition of antigen and labelled antibody was performed separately. Whereas the direct assay incorporated a simultaneous incubation of antigen and labelled antibody.

In the initial investigations an enzyme labelled secondary anti-mouse-HRP was used to show the assay was working. Later this was replaced with mouse anti-CK-MB coated paramagnetic particles, with incubation on the electromagnet with studs in place to focus the field under the 2 following conditions:
1. 5 minute incubation period in a permanent field (500 mA/282 mT)
2. 5 minute incubation period - magnet 1 min on, 1 min off for 5 mins (500 mA/282 mT).

### Results

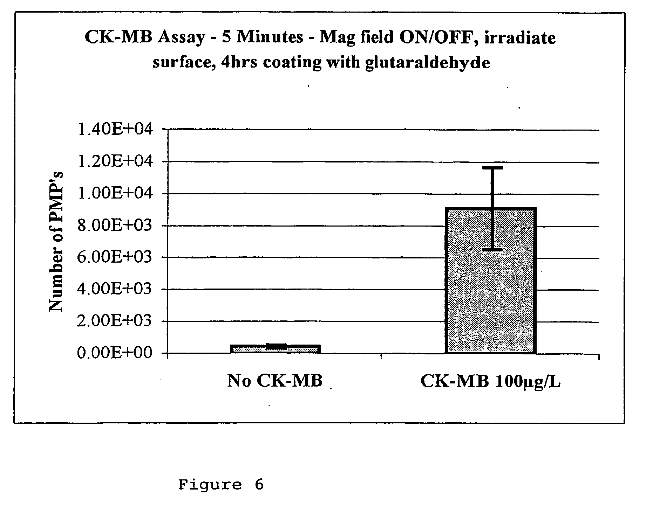

The combination of the following steps/conditions produced a good differential response (compared to control) as shown in Figure 6.
- Coating: Irradiate sticks for 15 minutes on UV illuminator with active surface towards the UV source. Coat with 25 µl goat anti-CKMB (1/1000) and 10 µl 1% glutaraldehyde. Incubate for 4 hours at room temperature.
- Blocking: 1 hour with 50 µl 1% BSA/ 1M glycine/ PBS.
- PMP's: Dilute CK-MB 1:1000 (100 µg/L) in 1% BSA/PBS/1M glycine. Mix 10 µl coated PMP's with 10 µl CK-MB. Antibody was cross-linked using triethanolamine
- Assay: Incubate on electromagnet: 1 minute on, 1 minute off, for 5 minutes. Max field (500 mA/282mT).

Wash with 2 x 2ml PBS, 1 x 1ml dH₂O. Allow to dry and read in the magnetometer.

### Example 3

### CRP Assay - using nanometer sized paramagnetic particles (Ferrofluid)

Nanometre sized paramagnetic particles were used in an assay system for measuring C-reactive protein (CRP). As paramagnetic particles could not be counted by microscopy an initial experiment was performed to investigate the magnetic susceptibility of the ferrofluid in the magnetometer. This was compared with the susceptibility of the micron sized paramagnetic particles. It was found that for a given mass of paramagnetic material the ferrofluid gives a greater response in the magnetometer.

### Assay development

Knowing that the ferrofluid could be detected in the magnetometer, the development of a 5-minute, competitive assay was investigated. The assay had to be competitive, as a labelled antigen was used. In the assay the anti-CRP was immobilized on the support material and sample antigen and labelled antigen competed for a limited number of antibody binding sites immobilized on the support material.

As with the CK-MB assay, a wide range of conditions for coating the support material was investigated to try and maximise the amount of protein immobilized. These included:
- Incubating overnight at 4°C.
- Incubating at room temperature for 4 hours.
- Irradiation of the solid phase for 15 minutes using a trans-illuminator prior to applying the protein.

All the above were with and without glutaraldehyde in the coating buffer and using a range of antibody dilutions: 1/200, 1/500 and 1/1000, 1/5000.

The assay was performed by mixing test antigen with labelled antigen (the CRP-ferrofluid). This was performed for 1/50 and 1/500 dilutions of a pooled serum with a high CRP concentration. The ferrofluid was diluted 1/3 for use. The 5-minute incubation on the electromagnet was performed in the presence of studs to focus the magnetic field at a field strength of 282 mT.

### Results

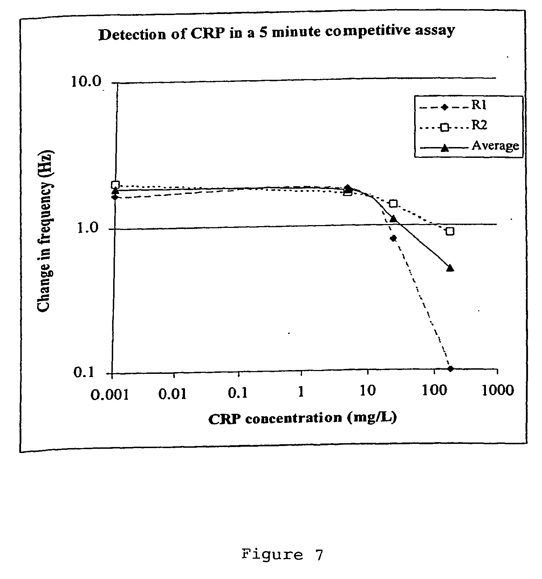

Of all the different combinations of assay conditions investigated, the conditions described below gave the best results. These results are shown in Figure 7. The assay was repeated on two separate days, the graph shows the response on the two days (R1 and R2) and a mean response. The conditions used in the assay were:
- Coating: Irradiate sticks for 15 minutes on UV illuminator with active surface towards the UV source. Dilute anti-CRP 1:500 in PBS. Apply 25 µl anti-CRP and 10 µl PBS. Incubated at room temperature for 4 hours.
- Blocking: Blocking in 1% BSA/ PBS for 1 hour at room temperature.
- Ferrofluid: Dilute CRP standards 1:500 in 1% BSA/PBS Mix 10 µl CRP-ferrofluid (diluted 1/3) with 10 µl CRP. Incubate on electromagnet for 5 minutes, perm field, maximum field strength (500 mA, 282 mT). Wash3x 2ml PBS. Read in magnetometer when dry.

### Example 4

### CK-MB Assay - quantitative measurement

This experiment was carried out to establish calibration curves for various concentrations of analyte, to provide quantitative results.

Polyester disks of 1.5 cm diameter were used. These were activated as follows:
- Dip in methanol for 1 minute and UV irradiate for 10 minutes.
- Wash in methanol.
- Dip in polymerised glutaraldehyde for 30 minutes (5 ml 5% glutaraldehyde, 500 µl 0.1M NaOH).
- Dip in methanol for 1 minute.

Dilute capture antibody (1/1000 to 5.56 µg/ml) was added to 0.1 M bicarbonate buffer pH 9.7 containing 2% methanol and 0.5% glutaraldehyde. This was applied (35 µl) to the disc. The disc was then incubated for 4 hours at room temperature, washed in PBS and blocked in 1% BSA/glycine/PBS for 1 hour.

Paramagnetic particles were prepared as follows:
- Dilute Goat CKMB (1/1000 to 5.73 µg/ml) in 0.1M Na phosphate buffer pH 5.8.
- Wash PMP 280 (10µl per sample) 2- 3X in the above buffer.
- Mix 30µl Goat CKMB + 10ul PMP (per disc), incubate 10 min RT on a slowly revolving Dynal Sample Mixer.
- Finally wash 3 times in PBS with 1% BSA = 0.1% Tween 20.

The following CKMB solutions were used:
1. 1/1000 (250ng/ml)
2. 1/2000 (125ng/ml)
3. 1/4000 (62.5ng/ml)
4. 1/8000 (31.25ng/ml)
5. 1/16000 (15.6ng/ml)
6. 1/32000 (7.8ng/ml)

0.9 ml of appropriately diluted CKMB was then injected into the reaction vessel, (control vessels receive PBS/BSA) and 100 µl of labelled PMPs (diluted 1/10 in PBS) were added into each reaction vessel (triplicate assays were performed). The solutions were mixed and the frequency was determined immediately (0 time). The magnetic field was applied using an upper and a lower magnet (either above or below the support surface), with each magnet being moved alternately towards or away from the support to apply the magnetic field. The frequency was determined every two minutes when the lower magnet was at the maximum distance from the support.

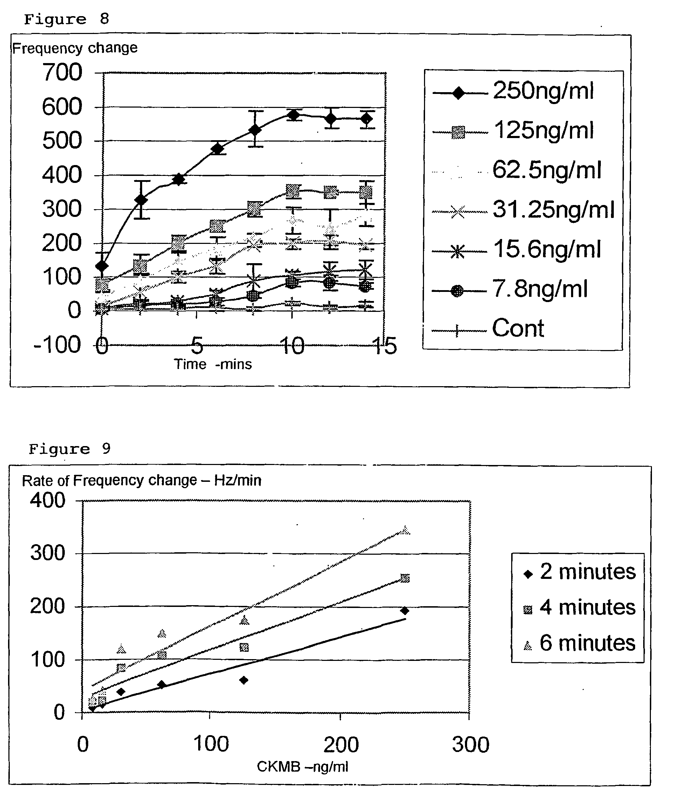

The change in frequency was determined according to the following equation:
Absolute difference of [(Mean frequency reading of sample) - (Mean frequency reading of blank)]. The results are shown in Figures 8 and 9.

## Claims

1. A method for obtaining kinetic measurements during a binding assay, comprising the steps of:
(i) bringing a target molecule into contact with its affinity partner to form a binding complex, wherein the target molecule is immobilised on a magnetic particle and the affinity partner is immobilised on a support material; and
(ii) determining the number of complexed magnetic particles during the assay, by exposing the complex to a magnetic field at regular time intervals during the assay and measuring the difference in the resonant frequency of a tuned circuit when the complex is exposed to the magnetic field and when it is not.

2. A method according to claim 1, wherein the support material is in a reaction vessel and the assay is carried out in liquid suspension.

3. A method according to claim 1 or claim 2, wherein the number of complexed magnetic particles is determined by a detection coil.

4. A method according to any preceding claim, wherein the target molecule is an antibody.

5. A method according to any preceding claim, wherein the affinity partner is an antibody or antigen.

6. A method according to claim 5, wherein the affinity partner is an antigen.

7. A method according to any preceding claim, wherein the affinity partner is immobilised to the support material via an intermediary antibody.

8. A method according to any of claims 1 to 3, wherein the target molecule is a polynucleotide.

9. A method according to claim 1 or claim 8, wherein the affinity partner is a polynucleotide.

10. A method according to any preceding claim, wherein the magnetic field is applied constantly.

11. A method according to any of claims 1 to 9, wherein the applied magnetic field is variable.

12. A method according to claim 11, wherein the magnetic field is varied by applying an external signal as a square, sine or sawtooth waveform.

13. A method according to claim 12, wherein the magnetic field is varied by applying a square waveform.

14. A method according to any preceding claim, wherein the magnetic field is provided by means of an electromagnet comprising metal studs to focus the magnetic field.

15. A method according to any of claims 1 to 13, wherein the magnetic field is provided by means of a coiled electromagnet.

16. A method according to claim 15, wherein a plurality of coiled electromagnets are used.

17. A method according to any preceding claim, wherein any non-bound magnetic particle is removed from the surface of the support material, by use of a magnet positioned above the surface of the support material.

## Patentansprüche

1. Verfahren zur Gewinnung kinetischer Messungen während eines Bindungstests, umfassend die Schritte:
(i) in Kontakt bringen eines Zielmoleküls mit seinem Affinitätspartner zur Bildung eines Bindungskomplexes, wobei das Zielmolekül auf einem magnetischen Partikel immobilisiert ist und der Affinitätspartner auf einem Trägermaterial immobilisiert ist; und
(ii) bestimmen der Anzahl komplexierter magnetischer Partikel während des Tests durch Aussetzung des Komplexes einem magnetischen Feld in regelmäßigen Zeitintervallen während des Tests und messen der Differenz der Resonanzfrequenz eines abgestimmten Schwingkreises, wenn der Komplex dem magnetischen Feld ausgesetzt ist und der Komplex nicht dem Magnetfeld ausgesetzt ist.

2. Verfahren nach Anspruch 1, wobei das Trägermaterial in einem Reaktionsgefäß vorliegt und der Test in flüssiger Suspension durchgeführt wird.

3. Verfahren nach Anspruch 1 oder Anspruch 2, wobei die Anzahl komplexierter Magnetpartikel durch eine Detektionsspule bestimmt wird.

4. Verfahren nach einem der vorhergehenden Ansprüche, wobei das Zielmolekül ein Antikörper ist.

5. Verfahren nach einem der vorhergehenden Ansprüche, wobei der Affinitätspartner ein Antikörper oder Antigen ist.

6. Verfahren nach Anspruch 5, wobei der Affinitätspartner ein Antigen ist.

7. Verfahren nach einem der vorhergehenden Ansprüche, wobei der Affinitätspartner auf dem Trägermaterial über einen Intermediär-Antikörper mobilisiert ist.

8. Verfahren nach einem der Ansprüche 1 bis 3, wobei das Zielmolekül ein Polynukleotid ist.

9. Verfahren nach Anspruch 1 oder Anspruch 8, wobei der Affinitätspartner ein Polynukleotid ist.

10. Verfahren nach einem der vorhergehenden Ansprüche, wobei das magnetische Feld konstant angelegt wird.

11. Verfahren nach einem der Ansprüche 1 bis 9, wobei das angelegte magnetische Feld veränderlich ist.

12. Verfahren nach Anspruch 11, wobei das magnetische Feld durch Anlegen eines externen Signals in Rechteck-, Sinus- oder Sägezahnwellenform veränderlich ist.

13. Verfahren nach Anspruch 12, wobei das magnetische Feld durch Anlegen einer Rechteckwellenform veränderlich ist.

14. Verfahren nach einem der vorhergehenden Ansprüche, wobei das magnetische Feld mittels eines Elektromagneten bereitgestellt wird, der Metallstifte zum fokussieren des magnetischen Felds umfasst.

15. Verfahren nach einem der Ansprüche 1 bis 13, wobei das magnetische Feld mittels eines Elektromagneten, der eine Spule aufweist, bereitgestellt wird.

16. Verfahren nach Anspruch 15, wobei eine Mehrzahl von Elektromagneten, die eine Spule aufweisen, verwendet werden.

17. Verfahren nach einem der vorhergehenden Ansprüche, wobei jegliche nicht gebundenen magnetischen Partikel von der Oberfläche des Trägermaterials durch einen Magneten entfernt werden, der über der Oberfläche des Trägermaterials angeordnet ist.

## Revendications

1. Procédé d'obtention de mesures cinétiques pendant un dosage de liaison, comprenant les étapes consistant à :
(i) amener une molécule cible en contact avec son partenaire d'affinité pour former un complexe de liaison, où la molécule cible est immobilisée sur une particule magnétique et le partenaire d'affinité est immobilisé sur un matériau de support ; et
(ii) déterminer le nombre de particules magnétiques complexées pendant le dosage, par exposition du complexe à un champ magnétique à des intervalles de temps réguliers pendant le dosage et mesure de la différence dans la fréquence de résonance d'un circuit syntonisé lorsque le complexe est exposé au champ magnétique et lorsqu'il ne l'est pas.

2. Procédé selon la revendication 1, dans lequel le matériau de support se trouve dans une cuve de réaction et le dosage est réalisé en suspension liquide.

3. Procédé selon la revendication 1 ou la revendication 2, dans lequel le nombre de particules magnétiques complexées est déterminé par une bobine de détection.

4. Procédé selon l'une quelconque des revendications précédentes, dans lequel la molécule cible est un anticorps.

5. Procédé selon l'une quelconque des revendications précédentes, dans lequel le partenaire d'affinité est un anticorps ou antigène.

6. Procédé selon la revendication 5, dans lequel le partenaire d'affinité est un antigène.

7. Procédé selon l'une quelconque des revendications précédentes, dans lequel le partenaire d'affinité est immobilisé sur le matériau de support via un anticorps intermédiaire.

8. Procédé selon l'une quelconque des revendications 1 à 3, dans lequel la molécule cible est un polynucléotide.

9. Procédé selon la revendication 1 ou la revendication 8, dans lequel le partenaire d'affinité est un polynucléotide.

10. Procédé selon l'une quelconque des revendications précédentes, dans lequel le champ magnétique est appliqué constamment.

11. Procédé selon l'une quelconque des revendications 1 à 9, dans lequel le champ magnétique appliqué est variable.

12. Procédé selon la revendication 11, dans lequel le champ magnétique est varié par application d'un signal externe en tant que forme d'onde carrée, sinusoïdale ou en dents de scie.

13. Procédé selon la revendication 12, dans lequel le champ magnétique est varié par application d'une forme d'onde carrée.

14. Procédé selon l'une quelconque des revendications précédentes, dans lequel le champ magnétique est fourni au moyen d'un électroaimant comprenant des goujons en métal pour focaliser le champ magnétique.

15. Procédé selon l'une quelconque des revendications 1 à 13, dans lequel le champ magnétique est fourni au moyen d'un électroaimant bobiné.

16. Procédé selon la revendication 15, dans lequel une pluralité d'électroaimants bobinés est utilisée.

17. Procédé selon l'une quelconque des revendications précédentes, dans lequel toute particule magnétique non liée est retirée de la surface du matériau de support, par utilisation d'un aimant positionné au-dessus de la surface du matériau de support.
